(19) Europäisches Patentamt
European Patent Office
Office européen des brevets

(11) **EP 4 036 691 A1**

(12) **EUROPEAN PATENT APPLICATION**

(43) Date of publication:
**03.08.2022 Bulletin 2022/31**

(21) Application number: **21170639.5**

(22) Date of filing: **27.04.2021**

(51) International Patent Classification (IPC):
**G06F 3/01** (2006.01)  **A61B 5/16** (2006.01)

(52) Cooperative Patent Classification (CPC):
**G06Q 10/06; G06V 20/52;** A61B 5/165

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.01.2021 LT 2021002**

(71) Applicant: **Vilniaus Gedimino technikos universitetas**
**10223 Vilnius (LT)**

(72) Inventors:
• **KAKLAUSKAS, Arturas**
  **08402 Vilnius (LT)**
• **BINKYTE-VELIENE, Arune**
  **11311 Vilnius (LT)**
• **UBARTE, Ieva**
  **03138 Vilnius (LT)**
• **BUCINSKAS, Vytautas**
  **10231 Vilnius (LT)**

(74) Representative: **Pranevicius, Gediminas**
**Law firm IP Forma**
**Uzupio g. 30**
**01203 Vilnius (LT)**

(54) **A METHOD FOR PERSONALIZED MANAGEMENT OF BUILDING SMART SPACE QUALITY AND ITS IMPLEMENTATION SYSTEM**

(57) The invention relates to a personalized quality management method for digital twin building smart spaces and a system for implementing thereof, which personalizes smart space quality parameters, such as indoor air quality, a level of lighting intensity and colors, smells, images, learning materials, music, pollution, based on the user's emotional, affective and physiological states, valence, excitement, workability (learning efficiency), interest and non-boredom (AFFECT) metrics. The method and system enable real-time maintenance of high user performance (learning efficiency), required interest and non-boredom by adapting smart space quality metrics to the user's AFFECT states. The PAD emotional state model, inverted U-curve theory, neuro-decision making and neuro-correlation tables are used to evaluate the impact of digital twin smart spaces on users, their response dynamics in real time, and to react to said response.

**Fig. 1**

**Description**

FIELD OF THE INVENTION

The invention relates to a method for personalized management of digital twin building's smart space quality and its implementation system. The customized management of quality control covers the lighting intensity and colors, scents, media, information, knowledge, data, activities, learning materials, games, music, videos, temperature, humidity, air pollution, vibrations and the like pertinent to the smart spaces. These are the matters of management that generate a customized, adaptive environment for users with consideration of the measurements of their emotional, affective and physiological states, valence, arousal, work productivity (learning effectiveness) as well as interest and non-boredom (AFFECT). The purpose of this invention is to develop an innovative method and system, which would analyze, customize and manage the quality control pertinent to the smart spaces of buildings or mobile workplaces according to user AFFECT states in an integrated manner, as a singular entity. Additionally, over the course of this process, various values (e.g. user perceived, hedonic, utilitarian) of building smart spaces are established based on the measurements that describe AFFECT states and other aspects regarding building spaces.

BACKGROUND OF THE INVENTION

Currently there are several known, technological solutions in the field of customized, quality control of smart spaces pertinent to buildings. These encompass the technical solution for establishing physiological parameters and determining the equipment that describes the parameters of human emotions. However, these do not directly include the proposed method for the customized, quality control of building smart spaces nor the equipment designated for its implementation.

Patent Application No. US20140288714A1 of the United States, "Intelligent Energy and Space Management" is the most advanced analogue to use for comparing this invention. Its shortcomings that our invention overcomes are the following:

- This U.S. Patent Application only analyzes physiological and emotional parameters. It does not include an integrated analysis of user emotional states (happiness, sadness, anger, fear, disgust and the like), affective states (non-boredom, interest, confusion and the like), physiological states as well as the levels of valence, arousal, work productivity (learning effectiveness) interest and non-boredom among users. Therefore this invention, which is presented here, helps to establish the state of a user considerably more accurately.
- The U.S. Patent Application does not employ a neuro-decision making table or a neuro-correlation table for compiling customized diagrams pertinent to the control of the microclimate of building facilities. Furthermore there are no compilations or analyses of all possible alternatives pertinent to the quality of building smart spaces covering the level of lighting intensity and colors, scents, media, information, knowledge, data, activities, learning materials, games, music, video, temperature, humidity, air pollution, vibrations and the like. Thus this invention helps to uphold the quality of customized, smart spaces of buildings much more accurately, including the microclimate of facilities as well.
- The U.S. Patent Application does not establish various values (e.g. user perceived, hedonic, utilitarian) of building smart spaces by applying a set of biometric and intelligent technologies.

SUMMARY OF THE INVENTION

The features that make the invention presented here exceptional and innovative are the following:

- This invention has the capacity to analyze, in an integrated manner, the emotional states (happy, sad, angry, frightened, disgusted and the like), affective states (bored, interested, confused and the like) and physiological states of users, their valence and levels of arousal, work productivity (learning effectiveness), interest and non-boredom (AFFECT data). Additionally, during this analysis, there is also consideration of seasonality, the day of the week and circadian rhythm. Thereby this invention helps to determine the state of a user much more accurately.
- This invention compiles neuro-decision making and neuro-correlation tables that exhibit the alternatives pertinent to the qualities of customized smart spaces of buildings. Then all the possible qualitative alternatives for building smart spaces undergo analyses. This is the reason our invention presented here helps to uphold the customized quality of building smart spaces much better.
- This invention serves to establish the various values (e.g. user perceived, hedonic, utilitarian) of building smart spaces by applying a set of biometric and intelligent technologies.

The method and system relevant to this invention permit assessing the influence on humans caused by the smart spaces pertinent to the digital twin buildings. This is accomplished with the PAD (Pleasure, Arousal and Dominance) emotional state model in the form of neuro-decision making and neuro-correlation tables, self-learning regression model equations, emotional and affective state management algorithms. Then the dynamics of user reactions in real time permit reacting adequately to the same. The interplay between the quality of building smart spaces and AFFECT is mutual. In other words, these two objects under research are resiliently interrelated, constituting components of a single object under research. The method and system are universal, which generate conditions for analyzing, acting and optimizing human AFFECT states with consideration of seasonality, the weekday and circadian rhythm.

Mehrabian and Russell (1974) were interested in characterizing and metering emotional states. To accomplish this, they developed a psychological model, known as the PAD emotional state model. There are three dimensions to the PAD emotional state model applied for characterizing all emotions-pleasure, arousal and dominance. The study of nonverbal communication, like body language in psychology, employs this PAD (pleasure, arousal, dominance) model (Mehrabian 2007).

The method and the system analyze various AFFECT data about users. These AFFECT data include user emotional states (excited, uplifted, happy, satisfied, vigilant, pleasant, calm, relaxed, fatigued, bored, depressed, sad, downhearted, nervous or tense) and user affective states (interestingness, disappointment, boredom or confusion). Additionally there are measurements on physiological states that include eye blinking, heart rhythm (frequency, strength, reverberation and arrhythmia), parameters of the thermal state of skin and their fluctuation rates, oscillations in spinal artery walls (sound, vibration), respiration rate and depth, muscle tension, dynamics of tension/relaxation (speed over time), galvanic skin response and brain waves (frequency, amplitude), etc. The level of user arousal is determined based on this data. Furthermore there are measurements of valence, arousal, work productivity (learning effectiveness) and states of interestingness and non-boredom. Such AFFECT data generate conditions within building or mobile workplaces smart spaces for feedback interactions with the AFFECT state of a user. The method this invention proposes along with the quality of a building's smart spaces and the AFFECT equipment that are required for this method's implementation allow modeling and optimizing the quality of a building's smart space and a user as a singular entity, i.e., an integrated system. Such modelling is performed by analyzing AFFECT and other data in the form of neuro-decision making and neuro-correlation tables. Thereby all possible alternatives for building smart spaces are established along with their respective impacts on a user by employing multicriteria analysis methods for an appropriate assessment. This assessment establishes the various values (e.g. user perceived, hedonic, utilitarian) of the alternatives, which aid rational decision-making.

The essence of this invention covers the method for the customized quality control of the digital twin building smart spaces as well as for its implementation system. This, along with the help of a set of user AFFECT wearable 4 and remote 5 sensors collects user AFFECT data and transfers them into the digital twin decision-making system (DSS). The DSS data are then processed and analyzed for establishing the AFFECT state of users. These data constitute the basis for the customization pertinent to the quality of building smart spaces.

The variables pertinent to the quality of customized, rational, building smart spaces (e.g., consisting of lighting levels, colors, scents, media, information, knowledge, data, activities, learning materials, games, music, videos, temperature, air pollution, vibrations) will fluctuate. These fluctuations by predetermined sizes and time intervals occur in real time in an integrated manner. This occurs with consideration of related levels of valence, arousal, work productivity, learning effectiveness, interest and non-boredom among users.

The sets of user AFFECT wearable 4 and remote 5 sensors consist of devices for gathering data on faces, voices, brain waves, eye pupils and blinking, signatures, strides, scents, analyses of printing keyboards, breathing frequencies, body temperatures, galvanic skin responses and others. The level of user arousal is determined based on this data. The performance of a quantitative analysis of the established variables involves employing neuro-decision making and neuro-correlation tables.

The assistance of multicriteria decision-making methods (e.g., INVAR), which employ neuro-decision making tables, are used for calculating various values (e.g. user perceived, hedonic, utilitarian) on building smart spaces in real time. Such values must fluctuate between preset minimum values (least satisfaction of user needs but resulting in a very low-cost exploitation of the facilities) and maximum values (greatest satisfaction of user needs but resulting in a very expensive exploitation of the facilities). The quality of a building's smart spaces remains stable so long as the value ranges between the cutoff limits of minimal and maximal values. However, once the quality goes beyond such limits, the building's smart spaces once again become customized according to the given, neuro-correlational, regression equations for work productivity (learning effectiveness) as well as the levels of interest and non-boredom. Neuro-decision making tables are employed for this purpose.

Neuro-correlations (correlation coefficients) are established in real time for numerous levels of quality pertinent to the smart spaces of buildings and many user AFFECT parameters. Use of the probability theory or statistical methods assist in establishing such real time statistical relationships between the levels of quality pertinent to building smart spaces and user AFFECT variables. Several correlation coefficients can be employed such as, for example, the Pearson linear correlation coefficient, Spearman rank correlation coefficient or others.

Set of devices 2 for the quality measure and customized control of smart spaces, as well as their data processing equipment can measure variables such as vibrations, air humidity, temperature, lighting and air pollution of the facilities, which correlate strongly with the AFFECT states of users. Set of user AFFECT wearable 4 and remote 5 sensors with data processing equipment establish user AFFECT states. This means AFFECT variables have a direct influence on the decision that establishes a customized smart spaces for housing facilities.

Once the chosen DIN regression equations for work productivity, interest, non-boredom, required affective states, positive and negative emotions are satisfied, the method of customized quality control pertinent to the smart spaces of digital twin buildings and this method's system of implementation have achieved their goals. Now they are capable of upholding existing parameters.

Assuming the chosen user's work productivity (learning effectiveness), interest, non-boredom, required affective states, positive and negative emotions (DIN) regression equations are not satisfied, then the customized quality control method pertinent to digital twin building smart spaces and its system of implementation have not achieved predetermined goals. Thus the AFFECT data continue being analyzed. Such an analysis further changes the parameters pertinent to the quality control of smart spaces in the pursuit for optimizing the state of a user, which, then, satisfies the DIN regression equation.

The arousal level based on the inverted "U" determine work productivity levels (Seyle 1975, McGrath 1976). The customization of the quality pertinent to the smart spaces of buildings directly influence the levels of user work productivity, their interest and non-boredom.

A smart database stores the measurements pertinent to rational, customized, smart spaces of buildings and pertinent to AFFECT. This smart database serves as the basis for calculating and supplementing the neuro-decision making and neuro-correlation tables, managing the quality control of the smart spaces pertinent to buildings as well as predicting and controlling the AFFECT metrics.

## BRIEF DESCRIPTION OF FIGURES

Fig. 1 presents the structure of the method for customized quality control pertinent to the smart spaces of the digital twin buildings and its system of implementation.

Fig. 2 presents the operational consistency of the method for customized quality control pertinent to the smart spaces of digital twin buildings and its system of implementation.

Fig. 3 presents a diagram displaying the managerial control of smart space parameters of a building by employing the set of devices for the quality measure and customized control of smart spaces, as well as their data processing equipment. Furthermore it establishes user AFFECT data by employing a set of user AFFECT wearable and remote sensors with data processing equipment.

## DETAILED DESCRIPTION OF THE INVENTION

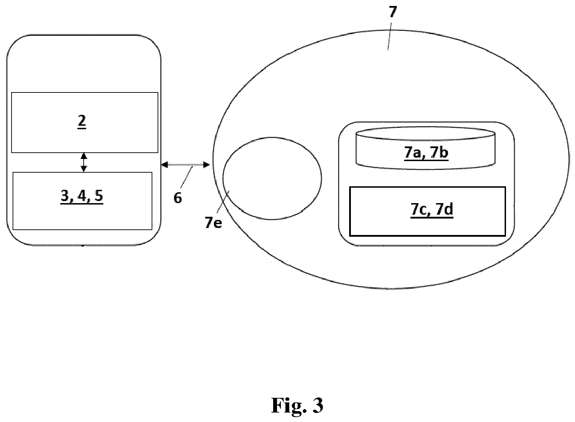

The method for customized quality control pertinent to smart spaces of digital, twin buildings and the system for its implementation operate sequentially, in stages, as presented next. The customized quality of building smart spaces covers lighting levels, colors, scents, media, information, knowledge, data, activities, learning materials, games, music, videos, temperature, humidity, air pollution, vibrations and, additionally, the user AFFECT data. The sequence of the invention's implementation is as follows (see Fig. 3):

1. Maintenance building or mobile workplaces smart spaces at specific geographic locations

2. Collecting data on temperature, humidity, air pollution, vibrations, lighting intensity and colors, scents, numbers of users and other features pertinent to the smart spaces of a building by employing a set of devices 2 for quality measurements and customized control of smart spaces, as well as for their data processing equipment

3-5. The collection of the AFFECT data pertinent to the emotional, affective and physiological states of users 3 along with their levels of valence, arousal, work productivity (learning effectiveness), interest and non-boredom occurs by employing the set of user AFFECT wearable 4 and remote 5 sensors with their data processing equipment.

6-7. Transferring the accumulated data via 5G or some other Internet network 6 to the digital twin decision-making system (SPS) 7 and saving the collected data in a local or in a cloud smart database 7a. The DSS consists of the smart database 7a and its management system (DMS) 7b along with the digital twin model base 7c with management system 7d and user interface 7e.

8. Selecting a desired type of service by the user 8.

9. Beginning a multicriteria analysis 9 by SPS based on the selected type of service by a user 3. Assessing and analyzing the gathered data considering circadian rhythm, seasonality and weekdays 9:

- Performing a multicriteria assessment on the quality parameters of a building's smart spaces considering the AFFECT data of the users within these facilities as well as seasonality, weekdays and circadian rhythm by applying neuro-decision making and neuro-correlation tables in the PAD emotional state model
- Establishing the most effective alternative from the obtained quality parameters pertinent to the potential smart spaces of a building
- Optimizing magnitude $x_{ij}$ pertinent to any indicator - rearranging the neuro-decision making table, whenever wanting to optimize magnitude $x_{ij}$ of any indicator, so magnitude $x_{ij\,opt}$ of the indicator under optimization becomes $x_{11}$, i.e., $x_{ij\,opt} = x_{11}$ and, then, seeking to establish the magnitude of $x_{11\text{-}R}$ for optimizing alternative $a_1$ so its quantity would be equal in the competitive market to the other alternatives $a_2$ - $a_n$ under comparison, undertaking a comprehensive analysis of all the indicator's positive and negative characteristics
- Calculating quantitative recommendations - showing possible improvements of indicator $x_i$ by percentile aiming for the indicator to be equal to the greatest possible magnitude for $x_{i\,max}$ as per the quantitative recommendation $i_{ij}$ for indicator $x_{ij}$
- Calculating qualitative recommendations - showing possible improvements of utility degree $N_j$ of indicator $x_i$, by percentile with qualitative recommendation $r_{ij}$ of indicator $x_{ij}$, when $x_{ij} = x_{i\,max}$. or, i.e., having $r_{ij}$ indicate the number of percentiles utility degree $N_j$ of alternative $a_j$ can be raised, assuming indicator magnitude $x_{ij}$ could be equal to the best magnitude of $x_{i\,max}$ for indicator $x_i$
- Calculating the number of cycles needed by approximation e seeking to establish the magnitude that indicator $x_{ij\,cycle\,e}$ should be for this alternative ($a_j$) to be the best among the alternatives ($a_1$-$a_n$) under comparison - the magnitude of indicator $x_{ij\,cycle\,e}$ is reduced/inccreased by amount e until the utility degree $N_{j\,e}$ of alternative ($a_j$) equals 100%

10-12. Compiling neuro-decision-making and neuro-correlation tables 10 based on the performed multicriteria analysis and the available data. Neuro-correlation tables are developed based on self-learning regression model equations. Emotional and affective state management algorithms well describe the operation of these equations. Calculations of values (e.g., user-perceived, hedonic, utilitarian) 11 and the determination of combinations of smart spaces parameters at a given time interval 12 are performed based on the neuro-decision-making tables above.

13. Saving 13 the results for the customization of the quality pertinent to the smart spaces of the digital twin buildings in the DSS smart database 7a.

14. Transferring 14 the personalization values into building' smart spaces.

15. Configuring, modifying and maintaining the quality of the building's smart spaces by their new personalized parameters 15.

The multicriteria decision-making method such as, for example, INVAR, which uses the neuro-decision making tables in real time, serves in aiding the calculations of the various values (e.g. user perceived, hedonic, utilitarian) in real time. These values should range between the assigned minimal value (the value that least satisfies user needs but proves very cheap for space exploitation) and the maximal value (the value that best satisfies user needs but proves very expensive for space exploitation). The quality of the smart spaces of relevant buildings remains stable so long as the value falls between the limits of the minimal and maximal values. However, once these value limits are surpassed, the parameters of the smart spaces are again changed according to the assigned neuro-correlation equations of regression model for statistical work productivity, interest, non-boredom required affective states, positive and negative emotions (DIN). Use of the neuro-decision-making tables serves this purpose.

One of the fundamental goals of this method is constantly upholding maximal work productivity (learning effectiveness), and desired interest and non-boredom, required affective states, positive and negative emotions level among users. The achievement of this maximal work productivity maintaining requires upholding the necessary level of arousal for the user under deliberation over the entire range of inverted U-curves (Seyle 1975, McGrath 1976) based on the established, customized equation of regression model of arousal for that specific user. This can be achieved by changing the parameters pertinent to the quality of the building's smart spaces. Such changes adjust the levels of user arousal and productivity according to inverted U-curve theories. A maximal level of productivity can be achieved as a marginal condition by regulating the level of the quality pertinent to the smart spaces of buildings. An unsuitable customization pertinent to the quality of the smart spaces of buildings will limit the maximal productivity of users, even if temporarily.

The work productivity (learning effectiveness) of a user depends on his/her level of arousal. Arousal ranges from 0 to 1. An equation of regression model regarding a determined level of arousal is established based on the obtained data pertinent to a customized level of arousal of some specific user and the quality of the building smart spaces. When there is no data existing pertinent to the arousal level of a user under deliberation and dependency of arousal on the quality of building smart spaces, then the equations of regression model relevant to the arousal of other, similar users are applied until the appropriate data has been accumulated. The regression analysis of quality control covers the level of lighting intensity (Light) and colors (Color), scents (Scent), information (Info), knowledge (Knowl), data (Data), music (Music), temperature (Temp), humidity (Humid), air pollution ($SO_2$, $KD_{2,5}$, $KD_{10}$, $NO_2$, CO, $O_3$), vibrations (Vibr), the error term ($\varepsilon$) and the like pertinent to the smart spaces. As example, a regression analysis is performed, which permits compiling a linear regression model (1) on arousal:

$$A = \beta_0 + \beta_{Humid} \cdot Humid + \beta_{Temp} \cdot Temp + \beta_{Light} \cdot Ligh + \ldots + \beta_{SO2} \cdot SO_2 + \beta_{KD2,5} \cdot KD_{2,5} +$$

$$+ \beta_{KD10} \cdot KD_{10} + \beta_{NO2} \cdot NO_2 + \beta_{CO} \cdot CO + \beta_{O3} \cdot O_3 + \ldots + \beta_{Col} \cdot Color + \beta_{Sc} \cdot Scent +$$

$$+ \beta_{Mu} \cdot Music + \beta_{Vibr} \cdot Vibr + \beta_{Info} \cdot Info + \beta_{Knowl} \cdot Knowl + \beta_{Data} \cdot Data + \varepsilon \qquad (1)$$

$\beta$ can have both positive and negative signs. This formula's practical operation is explained a little bit below by the example of the change arousal ($A_r$) algorithm.

The customized regression models compiled on a user pertinent to his/her arousal permit concluding that this variable was statistically significant with $p < 0.05$. Meanwhile the independent variables that comprise the models explain, to a large part, the dispersion of the magnitude pertinent to the dependent variable, in this case, to arousal.

The quality of a building's smart spaces must maintain desirable comfort for a user. Sanitation standards along with a user's interestingness (e.g. $I > 0.1$; I ranges from 0 to 1) and his/her non-boredom (e.g. $N > 0.1$; N ranges from 0 to 1) indicate whether or not the comfort of the surroundings have been assured at a workplace, at least minimally. An equation of regression model establishing a level of arousal is based on the obtained data customized for some specific user. Equations of regression model establishing the level of non-boredom and of interest are based on the obtained data customized for some specific user pertinent to his/her non-boredom and interest as well as on the quality of a building's smart spaces. Assuming there is no available data on the dependency of the non-boredom or of interest on the quality of the space pertinent to a user under deliberation, then equations of regression model of non-boredom and of interest taken from other, similar users are applied until the appropriate data is accumulated. As example, performance of a regression analysis permits compiling linear regression models on interest (I) and on non-boredom (N) (2 and 3):

$$I = \beta_{0I} + \beta_{I\,Humid} \cdot Humid + \beta_{I\,Temp} \cdot Temp + \beta_{I\,Light} \cdot Ligh + \ldots + \beta_{I\,SO2} \cdot SO_2 + \beta_{I\,KD2,5} \cdot$$

$$KD_{2,5} + \beta_{I\,KD10} \cdot KD_{10} + \beta_{I\,NO2} \cdot NO_2 + \beta_{I\,CO} \cdot CO + \beta_{I\,O3} \cdot O_3 + \ldots + \beta_{I\,Col} \cdot Color + \beta_{I\,Sc} \cdot$$

$$Scent + \beta_{I\,Mu} \cdot Music + \beta_{I\,Vibr} \cdot Vibr + \beta_{I\,Info} \cdot Info + \beta_{I\,Knowl} Knowl + \beta_{I\,Data} \cdot Data + \varepsilon \qquad (2)$$

$$N = \beta_{0N} + \beta_{N\,Humid} \cdot Humid + \beta_{N\,Temp} \cdot Temp + \beta_{N\,Light} \cdot Ligh + \ldots + \beta_{N\,SO2} \cdot SO_2 + \beta_{N}$$

$$_{KD2,5} \cdot KD_{2,5} + \beta_{N\,KD10} \cdot KD_{10} + \beta_{N\,NO2} \cdot NO_2 + \beta_{N\,CO} \cdot CO + \beta_{N\,O3} \cdot O_3 + \ldots + \beta_{N\,Col} \cdot Color$$

$$+ \beta_{N\,Sc} \cdot Scent + \beta_{N\,Mu} \cdot Music + \beta_{N\,Vibr} \cdot Vibr + \beta_{N\,Info} \cdot Info + \beta_{N\,Knowl} \cdot Knowl + \beta_{N\,Data} \cdot$$

$$Data + \varepsilon \qquad (3)$$

The customized regression models compiled on a user pertinent to his/her non-boredom and interest permit concluding that these have significant influence at $p < 0.05$. Meanwhile the independent variables that comprise the models explain, to a large part, the dispersion of the magnitude pertinent to the dependent variables, in this case, to interest and non-boredom.

Over time, the regression models' equations based on accumulated data and applied machine-learning algorithms are self-learning. In this case, we applied self-learning regression models.

The desirable quality of building smart spaces is maintained based on the customized equations of regression model of arousal, non-boredom, interest, required affective states, positive and negative emotions pertinent to a specific user, when seeking to maintain a user's maximum work productivity as per the inverted range of U-curves along with his/her desired levels of non-boredom and interest.

Three features describe the technical results of this invention:

- Since this invention analyzes users in an integrated manner pertinent to their emotional states (happy, sad, angry, frightened, disgusted and the like), affective states (bored, interested, confused and the like) and physiological states along with their valence and arousal (AFFECT data). Additionally it establishes the levels of the work productivity (learning effectiveness), interestingness and non-boredom of these same users, plus, with consideration to seasonality, weekday and circadian rhythm. Thereby this invention is an aid for establishing user states more accurately, to a significant degree.

- This invention involves the use of neuro-decision making and neuro-correlation tables when compiling all the possible alternatives pertinent to qualitative, customized building smart spaces. The aspects of customized quality can include level of lighting intensity and colors, scents, media, information, knowledge, data, activities, learning materials, games, music, videos, temperature, humidity, air pollution, brainwaves, vibrations and the like. A compilation of all possible alternatives pertinent to a building's smart spaces is analyzed; thereby this invention assists in retaining the desirable parameters to the customized quality of the building's smart spaces more accurately, to a significant degree.

- This invention applies multicriteria analysis methods (e.g. INVAR), biometric and intelligent technologies comprehensively for establishing various values (e.g. user perceived, hedonic, utilitarian) of a building's smart spaces. Thereby the establishment of the value pertinent to a building's smart spaces with the assistance of this invention proves to be far more accurate for users, to a significant degree.

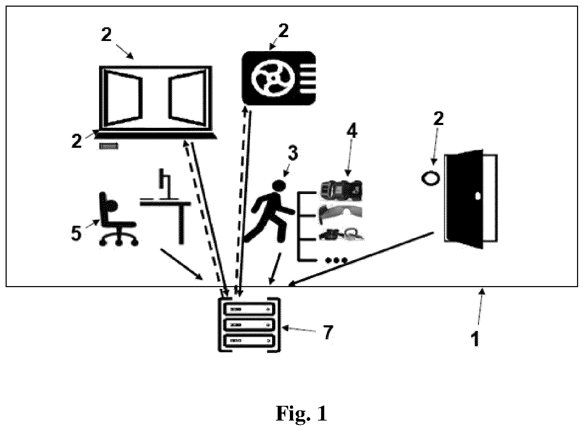

Figure 1 illustrates the customized method of quality control for the building or mobile workplaces smart spaces 1 and the structure of its implementation system. The digital twin decision-making system (DSS) 7 gathers AFFECT data from a set of user AFFECT wearable 4 and remote 5 sensors with their data processing equipment pertinent to studied user 3 by employing this invention's method. Meanwhile the system continuously measures the parameters pertinent to the quality of a building's smart spaces under existing conditions. Both the method and the system are designated for customizing the quality of a building's smart spaces in the pursuit of maximally satisfying user needs. The customized temperature, humidity, air pollution, vibrations level of lighting intensity and colors, scents, views, information, knowledge, data, activities, learning materials, games, music, videos, numbers of users and other features are established for the smart spaces of a building. This is accomplished by employing the set of devices 2 for the quality measure and customized control of smart spaces, as well as their data processing equipment. In other words, the set of devices 2 for the quality measure and customized control of smart spaces, as well as their data processing equipment are employed to adjust and measure internal conditions pertinent to the quality control of smart spaces. The collection of the AFFECT data pertinent to the emotional, affective and physiological states of users along with their levels of valence, arousal, work productivity (learning effectiveness), interest and non-boredom occurs by employing the set of user AFFECT wearable 4 and remote 5 sensors with their data processing equipment. Data taken from the set of user AFFECT wearable 4 and remote 5 sensors and from the set of devices 2 for the quality measure and customized control of smart spaces, as well as their data processing equipment transfer over to digital twin decision-making system (DSS) 7. This, in turn, models and optimizes desired quality pertinent to the digital, twin, smart spaces. This way the AFFECT data generate the conditions for the building's smart spaces to interact with some user AFFECT state is enabling feedback. The method we propose here along with the system required for implementing such building customized smart spaces and AFFECT data permits modeling and optimizing the parameters pertinent to the quality of these building smart spaces and the user as one entity, as a singular system. The DSS receives and analyzes data from various digital, twin, smart spaces located at different geographical locations: in cities and countries and on continents. Nonetheless, the quality of every smart space is adapted to every user 3. Furthermore the recommendations provided pertinent to the quality control of the different, customized building's smart spaces 1 are with consideration of a user's AFFECT states as well as with the weekday, seasonality, circadian rhythm and geographic location.

Neuro-correlation tables are developed based on self-learning regression model equations. Emotional and affective state management algorithms well describe the operation of these equations. The practical operation of formula one is explained below by the arousal ($A_r$) management algorithm.

Measurements are constantly taken on the real-time of arousal ($A_r$) upon request from a user. Every few days, over the course of such a measurement, the amounts of arousal are established for each, specific user at minimal ($A_{min}$), average ($A_a$) and maximal ($A_{min}$) levels. Analogically the interval of arousal for a specific user over the past several days is divided into equal proportions of low ($A_{min} \leq A_r < A_{1/3}$), average ($A_{1/3} \leq A_r < A_{2/3}$) and high ($A_{2/3} \leq A_r \leq A_{max}$) levels of arousal.

Facilities managers, users and/or other interested groups determine initial, minimal and maximal values pertinent to the quality of the building's smart spaces involving, e.g., the intensity of lighting and its colors, aromas, temperature, humidity and air pollution. Additionally a user is able to make an advance selection pertinent to the regulatory components for managing the quality of the smart spaces of interest to him/her, based on the Lego principle. For example, certain users may not be interested in the possibilities for having scents or vibrations.

The following corrections are made pertinent to the quality of building smart spaces under conditions when the level of user arousal ($A_{2/3} \leq A_r \leq A_{max}$) exceeds an average level considering that same user's priorities:

- The level of pollution (e.g., hard particles, aerosols, CO, $CO_2$, $NO_x$, NO, $NO_2$) is regularly reduced by 10 mg/m$^3$. Assuming the level of arousal does not drop to the rate as prescribed to an average arousal rate while reducing pollution to the level specified by the user, then proceed to the next point, leaving the pollution level pre-selected by the user.

- Change the room lighting using colors that reduce arousal (e.g., green, blue, violet, white) while considering the user's preferences indicated on the questionnaire provided prior to the experiment. Assuming the arousal level does not drop to an average level as the room's lighting changes using one color after another, then proceed to the next point, leaving the color pre-selected by the user.
- The initial intensity of lighting is set at 500 lux (or lx). When a specific user user's real-time of arousal is greater than average ($A_{2/3} < A_r$), then the intensity of the lighting is uniformly reduced down to 200 lx or down to the minimal limit that sanitation standards define. Assuming the level of arousal does not drop to an average rate, then proceed to the next point, leaving the light intensity level pre-selected by the user.
- Increase or decrease the humidity of the facilities by increments of 1%. At first, reduce the humidity of the facilities to the lower limit as indicated by sanitation standards to reach an average level of arousal. Assuming arousal does not drop down to an average level, then increase the humidity of the facilities incrementally to the limit indicated by sanitation standards. Assuming the arousal level still does not drop to an average level, then proceed to the next point, leaving the humidity level pre-selected by the user.
- Raise the room temperature by 0.5 C° to the upper limit an indicated by sanitation standards. Assuming arousal does not drop to an average level, then proceed to the next point, leaving the temperature pre-selected by the user.
- The needed views, information, news, data, activities, learning materials, games, music, videos, vibrations and such presented are those that will decrease a user/user's arousal. Assuming arousal does not drop to an average level, then proceed to the next point.
- Play calming music, i.e., music that excites the parasympathetic nervous system. The musical expressions for achieving such an affect include a minor harmony for lowering blood pressure; a soft, low volume for slowing down the breathing rate and the pulse and an unaccented, slow rhythm that narrows eye pupils accompanied by a smoothly flowing melody. Assuming the level of arousal does not drop to average while such music is playing, then proceed to employ the arousal regulatory recommendations subsystem.

Continue performing all these actions until an average level of arousal ($A_{1/3} \leq A_r < A_{2/3}$) is achieved.

For insufficient, less than average levels of arousal ($A_r < A_{1/3}$), perform the following qualitative changes pertinent to these building smart spaces:

- Change room colors to arousing colors, such as red, orange, yellow and the like. The user selects the priority of color. Assuming the arousal is insufficient to raise the level of arousal to average, change the room lighting colors, one by one. Assuming this still does not arouse arousal to an average level, then proceed to the next point, leaving the color pre-selected by the user.
- The initial intensity of lighting is set at 500 lux (or lx). When a specific user user's real-time of arousal is lower than average ($A_r < A_{1/3}$), then the intensity of the lighting is uniformly increased to 1000 lx or up to the upper limit that sanitation standards define. Assuming arousal is still not aroused to an average level, then proceed to the next point, leaving the light intensity level pre-selected by the user.
- Raise or lower the humidity level of the facilities by increments of 1%. At first, lower the humidity level of the facilities to the lower limit indicated by sanitation standards seeking to arouse arousal to an average level. Assuming arousal is not aroused up to an average level, then raise the humidity in the facilities to the upper limit indicated by sanitation standards. Assuming arousal is still not aroused to an average level, then proceed to the next point, leaving the humidity level pre-selected by the user.
- Lower room temperature by 0.5 C° to the lowest limit indicated by sanitation standards seeking to arouse arousal to an average level. Assuming arousal is not aroused to an average level, then proceed to the next point, leaving the temperature pre-selected by the user.
- The needed views, information, news, data, activities, learning materials, games, music, videos, vibrations and such presented are those that will increase a user/user's arousal. Assuming arousal is still not aroused to an average level, then proceed to the next point.
- Excite the sympathetic nervous system, which is permissible by using music activation. The musical expressions that can be employed include cohesive harmony for increasing blood pressure, dissonances for increasing respiration and pulse rates, accents for dilating the eye pupils, dotted rhythms, pronounced pulsations, sudden melodic leaps and staccato strokes. Assuming there is still no arousal of arousal while the music is playing, then proceed to employ the arousal regulatory recommendations subsystem.

Repeat all these actions until an average level of arousal ($A_{1/3} \leq A_r < A_{2/3}$) is achieved.

Whenever there is a lower than average level of interest ($I_v < I_r$) and lack of boredom ($N_v < N_r$), then for a user/user are present activities, information, news, data, views, learning materials, vibrations, music and the like, one following another or ordered in a way that the user/user had established, however, with consideration of user priorities. Those displayed are intended to increase interest and decrease boredom. All this is performed by applying affective computing. It is also possible to apply the arousal regulatory recommendations subsystem, which proposes various possible actions for increasing interest and lessening boredom. Such examples can include having a short coffee break, interacting with colleagues of interest or engaging in better-liked activities. Continue performing all these actions until a greater than average level of interestingness ($I_v < I_e$) and non-boredom ($N_v < N_e$) as well is achieved. The bases for establishing the average levels of interestingness ($I_v$) and non-boredom ($N_v$) consist of statistical data existing about each user. These numbers may fluctuate over time.

Assuming a user has neither a desired positive nor a negative emotional state, affective attitude, first, a user's priorities are taken into consideration. Then, either to increase or decrease that particular user's affective states, positive or negative emotions, there is a customization of lighting intensity and colors, scents, information, news, data, views, learning materials, vibrations, music, videos and such, one after another or in the order already established by the user according to his/her desires. This is all accomplished by the application of affective computing. It can also be accomplished by applying the arousal regulatory recommendations subsystem, which proposes various possibilities for increasing or decreasing a user's affective states, positive or negative emotions. All actions are repeated until the desired level of affective states, positive or negative emotions is achieved.

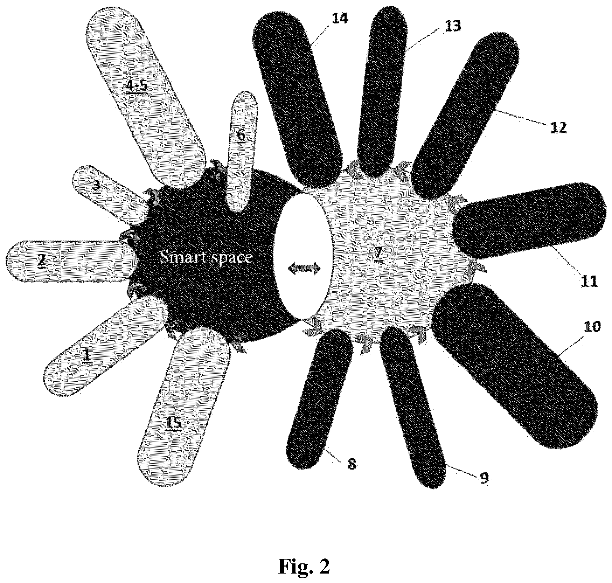

Fig. 2 illustrates the sequential operations pertinent to the customized, quality control method and its implementation system for digital, twin building, smart spaces.

The customized lighting level, color, scent, view, information, knowledge, data, activities, learning materials, games, music, video, vibrations, temperature, humidity and level of air pollution are established for building smart spaces 1 by employing the set of devices 2 for the quality measure and customized control of smart spaces, as well as their data processing equipment. Meanwhile the measurements of the emotional, affective and physiological states of users along with the levels of their valence, arousal, work productivity (learning effectiveness), interest and non-boredom (AFFECT) are established by employing the set of user AFFECT wearable 4 and remote 5 sensors along with data processing equipment (see Fig. 3). The set of user AFFECT wearable 4 and remote 5 sensors along with the set of devices 2 for the quality measure and customized control of smart spaces, as well as their data processing equipment interacts with digital twin decision-making system (DSS) 7 via the 5G or other network 6 and user interface 7e. The DSS consists of the smart database 7a and its management system (DMS) 7b along with the digital twin model base 7c with management system 7d. Furthermore it interacts with user interface 7e.

Digital twin decision-making system 7 determine combinations of smart spaces parameters at a given time interval 12 by employing the data from the set of devices 2 for the quality measure and customized control of smart spaces, as well as their data processing equipment.

Table 1 lists the types of services 8 provided.

Table 1

| Service type (ST) | Service type description (STD) | Service parameters (SP) and actions assuring them |
|---|---|---|
| Establishing and maintaining optimal parameters for the quality of a building's customized smart spaces (1.1) | Switching quality parameters of building smart spaces with the set of devices for the quality measure and customized control of smart spaces by employing a PAD emotional state model and the neuro-decision making and neuro correlations tables for compiling all possible alternatives of quality pertinent to a building's smart spaces, then assessing their impact on a user with multicriteria analysis methods - this is in pursuit of optimizing the quality of a building's smart spaces according to the user AFFECT data by the user's work productivity (learning effectiveness) and levels of interest and non-boredom. The user determines his/her desired work productivity (learning effectiveness) and levels of interest and non-boredom by his/herself (1.2) | Lighting level, color, scents, views, information, knowledge, data, activities, learning materials, games, music, videos, vibrations, temperature, humidity, air pollution and the like pertinent to a building's smart spaces (SP). The set of devices for the quality measure and customized control of smart spaces interact with DSS over the 5G or other network in pursuit of establishing and maintaining optimal parameters for the quality of a building's customized smart spaces (1.3) |
| ↕ | ↕ | ↕ |

(continued)

| Service type (ST) | Service type description (STD) | Service parameters (SP) and actions assuring them |
| --- | --- | --- |
| Establishing and upholding required user AFFECT states (2.1) | Upholding required work productivity (learning effectiveness) of users and the levels of their positive and negative emotions, affective states, interest and non-boredom (as established by sets of user AFFECT wearable and remote sensors with their data processing equipment) by changing the quality parameters pertinent to the smart spaces (2.2) | User work productivity (learning effectiveness) and levels of positive and negative emotions, affective states, interestingness and non-boredom. User AFFECT wearable and remote sensors with their data processing equipment provide these parameters data via the 5G or other network to the digital twin decision-making system (DSS). DSS and the set of devices for the quality measure and customized control of smart spaces, based on the patent algorithm, maintain the established customized levels of lighting intensity and colors, scents, media, information, knowledge, data, activities, learning materials, games, music, videos, vibrations, temperature, humidity, air pollution and music pertinent to the smart spaces (2.3) |

The method of quality control pertinent to the customized, digital, twin building, smart spaces along with its implementation system includes establishing the optimal parameters required to uphold the quality of the customized smart spaces of a building. Furthermore the optimal AFFECT state of a user must be established and then maintained according to a service type description (STD) and further, as per a categorization by service parameters (SP) and actions. The parameters of the user AFFECT states pertinent to certain changes in the quality of a smart space transfer into the set of devices 2 for the quality measure and customized control of smart spaces, as well as their data processing equipment in the pursuit of optimizing the information regarding the quality parameters of a specific, smart space in a specific building.

The establishment of appropriate quality parameters for a smart space (see Table 1, Point 1.1) involves obtaining data from the set of devices 2 for the quality measure and customized control of smart spaces of a specific building. These data then transfer into the digital twin model base 7c. The obtained data is next processed with consideration to the user's AFFECT state prior to forwarding them to the set of devices 2 for the quality measure and customized control of smart spaces, as well as their data processing equipment. This way, the parameters pertinent to the quality of a smart space are established with consideration of a user's 3 levels of work productivity (learning effectiveness), interest and non-boredom. The set of devices 2 for the quality measure and customized control of smart spaces interact with DSS over the 5G or other network 6 in pursuit of establishing and maintaining optimal parameters for the quality of a building's customized smart spaces.

The effort to establish and uphold a required optimal AFFECT state for a user (see Table 1, Point 2.1) involves employing the set of user AFFECT wearable 4 and remote 5 sensors and the devices with data processing equipment. In this instance, a required optimal user's levels of work productivity (learning effectiveness), interest and non-boredom are changed, controlled and upheld by changing the parameters pertinent to the quality of the smart spaces. The set of user AFFECT wearable 4 and remote 5 sensors along with their data processing equipment transmit data via 5G or other network 6 to the smart database 7a of the digital twin decision-making system (DSS) 7. DSS 7 normalizing and analyzing data, control commands are formed. The DSS 7, based on a patent algorithm and the computer's data, controls the set of devices 2 for the quality measure and customized control of smart spaces. These are the devices that assure the maintenance of an established customized level of lighting intensity and colors, scents, media, information, knowledge, data, activities, learning materials, games, music, videos, vibrations, temperature, humidity, air pollution and the like at the building or mobile workplaces smart spaces 1.

A user is able to select one of two service types at any given time: "establishing and maintaining optimal parameters for the quality of a building's customized smart spaces" or "establishing and upholding required user AFFECT states". Then a user registers a customized type of service for the specific smart space of a particular building. Then the DSS 7 transmits customized data on a user's AFFECT state and the results of calculations to a specific smart space of a particular building for devices 2 for the quality measure customized control of smart areas. The DSS 7 employs data on a user's AFFECT state and the results of calculations obtained from the set of user AFFECT wearable 4 and remote 5 sensors that establishes work productivity (learning effectiveness), interest and non-boredom. Finally, the DSS 7 issues commands for improving a user's work productivity (learning effectiveness), interest and non-boredom or evoke desired emotions and affective states by changing the parameters pertinent to the quality of a smart space.

The arousal regulatory recommendations subsystem, when aiming to reduce a user's arousal, displays on the computer screen calming views and colors and plays calming music and videos. The arousal regulatory recommendations subsystem, when aiming to arouse a user's arousal, provides on the computer either more interesting activities or learning materials; predominately negative information/reporting; energizing views, colors and scents; arousing music and videos and action movies. Each user assigns his/her own, personal priorities for all these activities. The arousal regulatory recommendations subsystem implements the priorities a user has assigned according to their order of priority.

The arousal regulatory recommendations subsystem provides suggestions for lowering or arousing arousal, excitement and boredom. When the level of arousal is below average, it suggests to the user to engage in activities prompting arousal. These can be using emotional words or statements; encouraging interesting albeit negative remembrances or energizing albeit positive remembrances and thoughts; engaging in physical activities and associating with friends and colleagues. When the level of arousal is greater than average, it suggests to the user to engage in activities prompting its reduction. Such actions can include reducing thoughts and remembrances, speaking calming words and statements, making a work or rest space comfortable, meditating, relaxing muscles progressively, reading pleasing books, swimming, enjoying relaxing pastimes, repeating words or thoughts like counting and breathing rhythmically from the diaphragm. Whenever there is a lower than average level of interest ($I_y < I_r$) and a lack of boredom ($N_v < N_r$), then the arousal regulatory recommendations subsystem can be applied upon consideration of a user's priorities. It proposes various possible actions for increasing interest and lessening boredom. Such actions can be enacted one after another, consequentially or in an order that the user/user establishes (e.g., taking a short coffee break, interacting with colleagues of interest or engaging in activities of greater interest).

**Claims**

1.  A method for personalized management of digital twin building's smart space quality, that uses a user's and building's space sensor data to decode the user's physiological and emotional parameters and building's space quality parameters in real-time, **characterized in that** the method, using the data from the user's (3) AFFECT portable (4) and remote (5) sensor sets, additionally digitally evaluates the user's (3) parameters, such as work productivity (learning effectiveness), interest, non-boredom, required affective states, positive and negative emotions (DIN), and presents this evaluation in the neuro-decision making and neuro-correlations tables by directing DIN data for setting and optimal personalized maintaining of quality parameters such as lighting level, colors, scents, views, music, vibrations, temperature and the levels of lighting, humidity, air pollution in smart spaces (1) of the building or mobile workplace.

2.  The method according to claim 1, **characterized in that** the DIN data is read from the user's (3) AFFECT portable (4) and remote (5) sensor sets in real time and this data is used to determine and control the optimal parameters of building smart spaces (1) with aim of quality measurement and personalized application and support control devices (2).

3.  The method according to claim 1, **characterized in that** the quality parameters of building smart spaces (1) are determined by their feedback on user's (3) DIN parameters and using neuro-decision making and neuro-correlation tables evaluating the impact of smart spaces (1) on user's (3) DIN parameters: work productivity (learning effectiveness), interest and non-boredom.

4.  The method according to any of the preceding claims, **characterized in that** the various building smart space values, such as user-perceived, hedonic, utilitarian, are determined on the basis of data taken from building (1) smart space quality measurement and personalized adaptation and maintenance control devices (2) and user's (3) AFFECT portable (4) and remote (5) sensor sets.

5.  A customized implementation system for controlling of quality of smart spaces of digital twin buildings, comprising devices for determining the user's emotional and physiological parameters and personalized management of building's smart space quality, **characterized in that** the system includes

    - the user's (3) AFFECT portable (4) and remote (5) sensor sets for integrated analysis of the user's emotional, affective and physiological states and for assessing the parameters related to the user's performance (learning efficiency), interest, non-boredom, required affective states, positive and negative emotions (DIN);
    - the personalized control devices (2) for the evaluation and management of the quality parameters of the building's smart spaces (1) according to the user-defined (3) DIN data;
    - a digital twin decision support system (SPS) (7) for collecting user's (3) emotional and physiological parameter data from the user's AFFECT portable (4) and remote (5) sensor sets and smart spaces (1) quality management devices (2) and for optimizing digital twin smart space quality parameters according to the user's AFFECT states.

6.  The system according to claim 5, **characterized in that** the digital twin decision-making system (SPS) (7) consists of a smart database (7a) and its management system (7b) and a digital twin model database (7c) and its management system (7d) interacting via a user's interface (7e) with user's (3) AFFECT portable (4) and remote (5) sensor sets and smart space quality measurement and customized control devices (2).

7.  The system according to claim 6, **characterized in that** the smart space quality parameters include lighting intensity and colors, scents, images, information, knowledge, data, works, learning materials, games, music, videos, temperature, humidity, air pollution, vibrations, and the like.

**Fig. 1**

**Fig. 2**

**Fig. 3**

Europäisches
Patentamt

European
Patent Office

Office européen
des brevets

# EUROPEAN SEARCH REPORT

Application Number

EP 21 17 0639

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/091515 A1 (THIEBERGER GIL [IL] ET AL) 17 April 2008 (2008-04-17) * abstract; claims 1-16; figures 1-52 * * paragraphs [0004] - [0010], [0064] - [0236] * | 1-7 | INV. G06F3/01 A61B5/16 |
| X | US 2019/387998 A1 (GARTEN ARIEL STEPHANIE [CA] ET AL) 26 December 2019 (2019-12-26) * abstract; claims 1-20; figures 1-20F * * paragraphs [0006] - [0059], [0084] - [1159] * | 1-7 | |
| X | US 2019/098725 A1 (SADWICK LAURENCE P [US] ET AL) 28 March 2019 (2019-03-28) * abstract; claim 1; figures 1-21 * * paragraphs [0025] - [0518] * | 1-7 | |
| X | US 2012/194648 A1 (HOFSHI NITTAI [IL]) 2 August 2012 (2012-08-02) * abstract; claims 1-16; figure 1 * * paragraphs [0004] - [0010], [0014] - [0035] * | 1-7 | TECHNICAL FIELDS SEARCHED (IPC) |
| X | US 2009/002178 A1 (GUDAY SHAI [US] ET AL) 1 January 2009 (2009-01-01) * abstract; claims 1-20; figures 1-10 * * paragraphs [0008], [0020] - [0047] * | 1-7 | A61B G06F |

The present search report has been drawn up for all claims

| Place of search | Date of completion of the search | Examiner |
|---|---|---|
| The Hague | 28 September 2021 | Streit, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 21 17 0639

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2021

| Patent document cited in search report | Publication date | Patent family member(s) | Publication date |
|---|---|---|---|
| US 2008091515 A1 | 17-04-2008 | NONE | |
| US 2019387998 A1 | 26-12-2019 | NONE | |
| US 2019098725 A1 | 28-03-2019 | NONE | |
| US 2012194648 A1 | 02-08-2012 | NONE | |
| US 2009002178 A1 | 01-01-2009 | NONE | |

EPO FORM P0459

For more details about this annex : see Official Journal of the European Patent Office, No. 12/82

**EP 4 036 691 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140288714 A1